# EUROPEAN PATENT SPECIFICATION

(11) **EP 0 661 315 B1**
(45) Date of publication and mention of the grant of the patent: **31.03.1999**
(21) Application number: 94120188.1
(22) Date of filing: 20.12.1994
(51) Int. Cl.: C08G 12/40, C09D 161/20, C08G 18/38, C07D 251/34

(54) **Extended isocyanurates for use as curing agents in coating compostitions**
Kettenverlängerte Isocyanurate zur Verwendung als Härtungsmittel in Beschichtungszusammensetzungen
Isocyanurates à chaîne allongée utilisables comme agent de durcissement dans des compositions de revêtement

(30) Priority: 03.01.1994 US 176608
(43) Date of publication of application: 05.07.1995
(73) Proprietor: BASF CORPORATION, Southfield, Michigan 48086-5009 (US)
(72) Inventor: Ohrbom, Walter H., Commerce Township, Michigan 48382 (US); Menovcik, Gregory G., Farmington Hills, Michigan 48334 (US)
(74) Representative: Münch, Volker, Dr.

(56) References cited:
- EP-A- 0 495 269
- EP-A- 0 622 387
- FR-A- 2 411 182
- US-A- 5 336 566

## Description

This invention relates to curable compositions, especially curable coating compositions, particularly acrylic compositions.

Polyurethane compositions based on triisocyanurates for coating and/or molding are well-known in the art. They provide a number of desirable characteristics such as resistance to solvent, salt, and other types of environmental attack.

However, these polyurethanes do suffer some disadvantages. An essential building block of these polyurethanes is the triisocyanurate. Triisocyanurates are expensive and difficult to handle. The NCO groups on the triisocyanurate are highly reactive, so they must be chemically blocked if it is desired to use the triisocyanurate in a one-pack coating composition. The use of chemical blocking groups further increases the expense of the material, results in increased VOC during cure, introduces an additional component into the composition that can have the potential for adverse side-effects such as yellowing, and necessitates a high heat curing temperature on the order of 150°C. If the NCO groups are not chemically blocked, the triisocyanurate must be utilized as one part of a two-pack composition. With such a composition, the highly-reactive triisocyanurate must be kept isolated from the surrounding environment and from the other component(s) of the composition until just before application to a substrate or mold, further increasing the expense and complexity of the process.

It has thus long been desired to produce a composition that exhibits the advantages of triisocyanurate-cured compositions having an optimum mix of characteristics as described above, but without having to use isocyanates groups as the functional groups for the curing reaction.

It is further desired that the coating composition demonstrate good sprayability when applied and good etch performance for the cured film.

According to the present invention, there is provided a coating composition obtained by the process of:
(a) reacting a tri-NCO-functional isocyanurate with a compound selected from the group consisting of monohydric alcohol, water, diol or diamine in a ration of 3:1 respectively so that on average, one of the isocyanate groups is reacted,
(b) reacting the isocyanurate obtained in step (a) with at least one compound to form a carbamate containing compound having the formula: 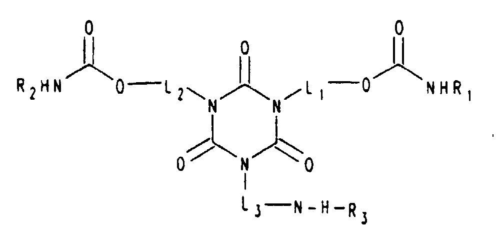 wherein
   L₁, L₂ and L₃ each independently represents a divalent linking group or a covalent bond, and
   R, and R₂ each independently represents H, alkyl, cycloalkyl, or aryl and R₃ is the residue resulting from the reaction of isocyanate and the compounds of (a) selected from the group consisting of monohydric alcohols, water, diols and diamines,
(c) reacting the component from step (b) with a component having a plurality of functional groups that are reactive with carbamate.

The component (a) used in the practice of the invention is formed by reacting a triisocyanurate with a compound selected from the group consisting of water, diamines, monohydric alcohols and diols, to provide an isocyanurate having one of the isocyanate groups defunctionalized or to provide a chain extended isocyanurate by reaction of one of the isocyanates. Preferably the isocyanate and water, alcohol, diamine or diol are reacted in a ratio of 3:1 respectively, so that on average, one of the isocyanate groups is reacted prior to formation of the carbamate component. Accordingly, the isocyanurate has only two isocyanate groups, on average, which are available for formation of the carbamate linkage.

For example when the triisocyanurate is reacted with a monohydric alcohol, one isocyanurate group reacts with the -OH group from the alcohol and is defunctionalized and unavailable for further reaction. The isocyanate functionality of the isocyanurate is in effect reduced from 3 to 2. When the triisocyanurate is reacted withwater, a diamine or diol, the isocyanurate is chain extended by first reacting one isocyanate group with the -OH group or -NH group and then subsequently reacting the OH or KH substituted isocyanurate with additional triisocyanurate, whereby the isocyanate functionality of the chain extended isocyanurate is increased from 3 to 4.

The isocyanurate obtained from step (a) is subsequently reacted with at least one compound to provide a carbamate containing compound having the formula 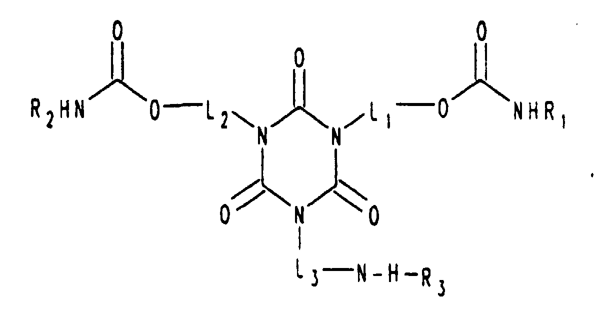 wherein
L₁, L₂, and L₃ each independently represents a linking group, and
R₁ and R₂ each independently represents H, alkyl, or cycloalkyl. R₃ is a residue resulting from the reaction of isocyanate and water, monohydric alcohol, diamine or diol.

The carbamate substituted compound is formed from the isocyanurate described in (a), having on average two reactive NCO groups, by reaction with a compound containing an isocyanate-reactive group and a carbamate group, e.g., a hydroxyalkyl carbamate such as hydroxypropyl carbamate or hydroxybutyl carbamate. Alternatively, the isocyanurate may be adducted with substituents that have the capability of forming carbamate groups after reaction with the isocyanurate compound is completed. For example, the isocyanurate can be reacted with a compound having a hydroxyl group and a cyclic carbonate group (e.g., the reaction product of glycidol and CO₂), and the cyclic carbonate groups then reacted with ammonia to form the carbamate functional groups.

The above-described NCO-functional isocyanurates are adducted with compounds containing a carbamate group or group that can be converted to carbamate and a group that is reactive with the -NCO functional group. Carbamate-containing compounds that can be adducted onto the NCO groups of an isocyanurate are preferably hydroxyalkyl carbamates such as hydroxypropyl carbamate or hydroxybutyl carbamate. Compounds containing groups that can be converted to carbamate and groups that are reactive with NCO include hydroxy-containing cyclic carbonate compounds convertible to carbamate by reaction with ammonia (e.g., the reaction product of glycidol and C0₂), monoglycidyl ethers (e.g., Cardura E®) convertible to carbamate by reaction with C0₂ and then ammonia, and monoglycidyl esters (e.g., the reaction product of a carboxylic acid and epichlorohydrin) convertible to carbamate by reaction with C0₂ and then ammonia, allyl alcohols where the alcohol group is reactive with NCO and the double bond can be converted to carbamate by reaction with peroxide, and vinyl esters where the ester group is reactive with NCO and the vinyl group can be converted to carbamate by reaction with peroxide, then C0₂, and then ammonia.

The composition of the divalent linking group L₁, L₂, and L₃ in the above formula may be controlled by the type of cyanuric ring compound or polyisocyanate chosen. The divalent linking groups L₁, L₂, and L₃ individually may be either an aliphatic (e.g., hexamethylene), cycloaliphatic (e.g., residue of isophorone diisocyanate or 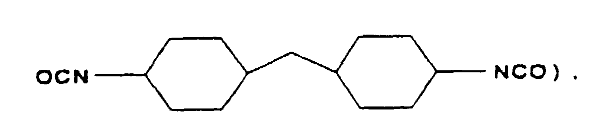 or aromatic group (e.g., residue of tetramethylxylylene diisocyanate), preferably up to 20 carbon atoms with terminal urethane, urea, or ester bonding to the substituent comprising the carbamate group. If resistance to solar degradation is desirable, then the divalent linking groups are preferably all aliphatic or cycloaliphatic. In a preferred embodiment, each of the L-groups independently represents a group having the formula -A-NH-COO-D- where A and D each represents a divalent linking group as described below.

In the above formulas, R₁ and R₂ each independently represents H, alkyl, preferably of 1 to 6 carbon atoms, or cycloalkyl, preferably up to 6 ring carbon atoms. It is to be understood that the terms alkyl and cycloalkyl are to include substituted alkyl and cycloalkyl, such as halogen-substituted alkyl or cycloalkyl. In a preferred embodiment, R₁, and R₂ are H, allowing for greater flexibility in the choice of the (a) component to react with the carbamate groups during cure. Substituents that will have an adverse impact on the properties of the cured material, however, are to be avoided. For example, ether linkages are thought to be susceptible to hydrolysis, and should be avoided in locations that would place the ether linkage in the crosslink matrix.

Although isocyanurates derived from cyanuric acid are useful in the present invention, isocyanurates formed by condensation of one or more types of diisocyanates, such as hexamethylene diisocyanate, or isophorone diisocyanate are preferred. Examples of preferred isocyanurates include the isocyanurate of hexamethylene diisocyanate, and the isocyanurate of isophorone diisocyanate. If light-fastness is not a critical requirement, then an aromatic isocyanurate such as the isocyanurate of 2,4-toluene diisocyanate may be used.

In a preferred embodiment of the invention, component (b) is derived from a trimerized diisocyanate, and has the formula (II): 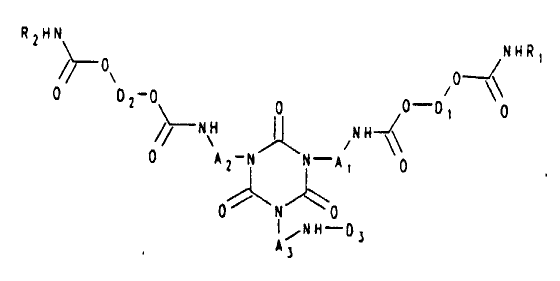 wherein
A₁, A₂, and A₃ each independently represents a divalent linking group, and
D₁, and D₂ each independently represents a divalent linking group. D₃ represents the residue resulting from the reaction between one isocyanate group and water, alcohol, diamine or diol.

In this formula, each A is a divalent linking group as is typically derived from the core of the diisocyanate used to form the isocyanurate, and may be either an aliphatic (e.g., hexamethylene), cycloaliphatic (e.g., residue of isophorone diisocyanate or 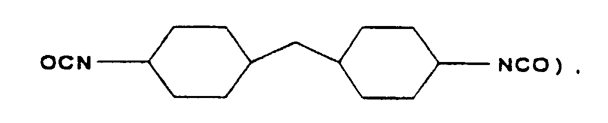 or aromatic group (e.g., residue of tetramethylxylylene diisocyanate), preferably up to 20 carbon atoms. D₁, and D₂ are divalent linking groups, preferably up to 20 carbon atoms, and are typically derived from the compounds having a carbamate group or carbamate-convertible group as described above. D₃ may be a urea linkage when the -NCO- is reacted with water or diamine or a urethane linkage when the -NCO- is reacted with diol.

The carbamate substituted compound (b) preferably has a molecular weight of 300 to 3000, preferably 450 to 1800. The equivalent weight per carbamate functional group can range from 100 to 1000, and preferably 150 to 600.

The component (c) used in the practice of the present invention has groups that are reactive with the carbamate groups on component (b). Such reactive groups include active methylol or methylalkoxy groups on aminoplast crosslinking agents or on other compounds such as phenol/formaldehyde adducts, isocyanate groups, siloxane groups, and anhydride groups. Examples of (c) compounds include melamine formaldehyde resin (including monomeric or polymeric melamine resin and partially or fully alkylated melamine resin), urea resins (e.g., methylol ureas such as urea formaldehyde resin, alkoxy ureas such as butylated urea formaldehyde resin), polyanhydrides (e.g., polysuccinic anhydride, copolymers of maleic anhydride), and polysiloxanes (e.g., trimethoxy siloxane). Aminoplast resin such as melamine formaldehyde resin or urea formaldehyde resin are especially preferred. Even more preferred are aminoplast resins where one or more of the amino nitrogens is substituted with a carbamate group for use in a process with a curing temperature below 150°C, as described in U.S. patent application Serial No. 07/965,510 entitled "Carbamate/Defunctionalized Aminoplast Curing for Polymer Compositions".

A solvent may optionally be utilized in the coating composition used according to the present invention. Although the formulation of the present invention may be utilized, for example, in the form of substantially solid powder, or a dispersion, it is often desirable that the formulation used in the present invention is in a substantially liquid state, which can be accomplished with the use of a solvent. Preferably the solvent is present in an amount effective to substantially solubilize both the (b) component and the (c) component. In general, the solvent can be any organic solvent and/or water. More preferably, the solvent is a polar aliphatic solvent or polar aromatic solvent. Still more preferably, the solvent is a ketone, ester, acetate, aprotic amide, aprotic sulfoxide, aprotic amine, and water. Examples of useful solvents include methyl ethyl ketone, methyl isobutyl ketone, m-amyl acetate, ethylene glycol butyl ether-acetate, propylene glycol monomethyl ether acetate, xylene, N-methylpyrrolidone, and blends of aromatic hydrocarbons.

The solvent may be present in the coating composition in an amount of from about 0.01 weight percent to about 99 weight percent, preferably from about 10 weight percent to about 60 weight percent, and more preferably from about 30 weight percent to about 50 weight percent.

The above-described coating compositions can be coated on the article by any of a number of techniques well-known in the art. These include, for example, spray coating, dip coating, roll coating, curtain coating, and the like. For automotive body panels, spray coating is preferred.

In one preferred embodiment, the composition of the invention is used as the clear coating composition over a pigmented basecoat as part of a composite color-plus-clear coating. Such composite coatings are popular for their depth of color and liquid glassy surface appearance. They have found particularly wide acceptance in the field of automotive coatings.

Pigmented basecoat compositions for such composite coatings are well-known in the art, and do not require explanation in detail herein. Polymers known in the art to be useful in basecoat compositions include acrylics, vinyls, polyurethanes, polycarbonates, polyesters, alkyds, and polysiloxanes. Preferred polymers include acrylics and polyurethanes. Basecoat polymers are preferably crosslinkable, and thus comprise one or more type of cross-linkable functional groups. Such groups include, for example, hydroxy, isocyanate, amine, epoxy, acrylate, vinyl, silane, and acetoacetate groups. These groups may be masked or blocked in such a way so that they are unblocked and available for the cross-linking reaction under the desired curing conditions, generally elevated temperatures. Useful cross-linkable functional groups include hydroxy, epoxy, acid, anhydride, silane, and acetoacetate groups. Preferred cross-linkable functional groups include hydroxy functional groups and amino functional groups.

Basecoat polymers may be self-cross-linkable, or may require a separate cross-linking agent that is reactive with the functional groups of the polymer. When the polymer comprises hydroxy functional groups, for example, the cross-linking agent may be an aminoplast resin, isocyanate and blocked isocyanates (including isocyanurates), and acid or anhydride functional cross-linking agents.

After the article is coated with the above-described layers according to the invention, the coated article is subjected to conditions so as to cure the coating layers. Although various methods of curing may be used, heat curing is preferred. Generally, heat curing is effected by exposing the coated article to elevated temperatures provided primarily by radiative heat sources. Curing temperatures will vary depending on the particular blocking groups used in the cross-linking agents, however they generally range between 82° and 144°, and are preferably between 110°C and 133°C. The curing time will vary depending on the blocking agents, and physical parameters such as the thickness of the layers, however, typical curing times range from 15 to 60 minutes.

Use of the isocyanurates reacted according to the present invention provides coatings with improved sprayability and improved appearance. Also, the cured films are less brittle and do not crack and demonstrate improved resistance to environmental etch.

The invention is further described in the following examples.

### Example 1

### Isocyanurate of Hexamethylene Diisocyanate Reacted with Hydroxy Acid

A reactor was charged with 368.7 parts xylene and 958.1 parts methyl isobutyl ketone and heated to reflux (122°C) under inert atmosphere. Once at reflux, the inert atmosphere was turned off and 24.2 parts of the refluxed solvent was removed. The reaction mixture was then cooled to 40°C under inert atmosphere and 1224 parts of isocyanurate of hexamethylene diisocyanate (N3300, from Miles Corporation, Pittsburgh, Pa), along with 4.0 parts dibutyl tin dilaurate were added. A slow purge of inert atmosphere was maintained for the rest of the reaction. The reaction mixture was then brought up to 60° C and 736 parts of 2,2-dimethyl-3-hydroxypropionic acid were added over 1 hour and 45 minutes. The reaction mixture was kept at 60°C for an additional 1 hour and 10 minutes. 63 parts of methyl amyl alcohol were then added over a 10 minute period. After the reaction mixture tested free of isocyanate by infrared spectrometry, 1217.0 parts methyl isobutyl ketone were added. The product had a solids content of 58.4% and a non-volatile acid equivalent weight of 333.6 g/eq.

### Example 2

A reactor was charged with 368 parts xylene and 626.2 parts methyl isobutyl ketone and heated to reflux: (122°) under inert atmosphere. Once at reflux, the inert atmosphere was turned off and 24.1 parts of the refluxed solvent was removed. The reaction mixture was then cooled to 55°C under inert atmosphere and 1411.3 parts of isocyanurate of isophorone diisocyanate (T1890, from HULS America, Inc., Piscataway, NJ), along with 4.2 parts dibutyl tin dilaurate were added. A slow purge of inert atmosphere was maintained for the rest of the reaction. 482.1 parts of 2,2-dimethyl-3-hydroxypropionic acid were added over 1 hour and 10 minutes, at a reaction temperature of between 51° to 57°C. The reaction mixture was kept at 54° to 60° for an additional 2 hours and 45 minutes. 45.7 parts of methyl amyl alcohol were then added over a 3 minute period. The product had a solids content of 58.7% and a non-volatile acid equivalent weight of 422 g/eq.

### Example 3

### Isocyanurate Having Carbamate Functional Group

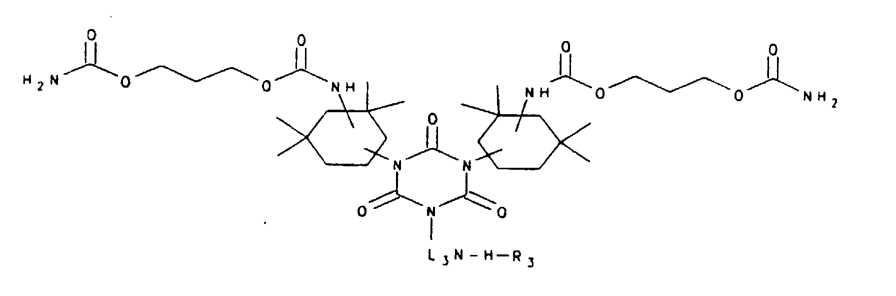

A reactor vessel under a nitrogen blanket was charged with 1047.0 g of the isocyanurate obtained from Example 2. (Based an the isocyanurate of isophorone diisocyanate, available from Huls America, Inc., Piscataway, NY), 4.2 g of dibutyltin dilaurate, and 356.7 g of the solvent propylene glycol monomethylether acetate. Temperature control was applied to the reaction mixture until a constant temperature of about 80°C was reached, and 381.1 g of hydroxypropyl carbamate was slowly added. The reaction was maintained until virtually all of the NCO had been consumed. At that point, additional solvent (337.8 g propylene glycol monomethylether acetate and 25.0 g n-butanol) was added to the mixture.

### Example 4 - Coating Example

### A clear coating composition was prepared having the following formulation:

| Ingredient | Parts by weight |
|---|---|
| Example 3 | 56.70 |
| Dispersion of partially-defunctionalized melamine formaldehyde resin* in xylene (51.9% non-volatiles) | 14.19 |
| Tinuvin 348B® | 6.84 |
| Tinuvin 123® | 0.42 |
| Dispersion of dodecylbenzene sulfonic acid (33.0% non-volatiles) | 0.84 |
| solvent blend (Exxate 800® and butanol) | 26.61 |

| | |
|---|---|
| *A melamine formaldehyde resin as described in U.S. patent application Serial No. 07/965,510. | |

This composition was then applied onto metal test panels that had been precoated with an unbaked high solids solvent-borne acrylic/melamine pigmented basecoat. The basecoat was applied to a primed metallic substrate test panel in two coats with a period of one minute in between coats to allow the first coat to flash dry. After the second basecoat was applied, the basecoat was flash dried, followed by application of the clear coat composition. The clearcoat was applied in two even coats with a flash between coats. The coated substrate was then allowed to dry for a short period, and then bake cured at 140°C for 30 minutes.

The panel was then placed on an outside exposure rack under severe etch-producing conditions for 4 weeks. The test panel coated with the coating of the invention (Example 2) had the most favorable etch rating of 1 on the General Motors etch evaluation scale. By comparison, a test panel coated with a state-of-the-art (prior art) clearcoat utilizing a hydroxy-functional acrylic polymer and an alkylated melamine-formaldehyde resin had an etch rating of 10 on the General Motors etch evaluation scale. This represents a significant advantage in the etch performance of the coating composition according to the invention.

## Claims

1. A coating composition obtained by the process of:
(a) reacting a tri-NCO-functional isocyanurate with a compound selected from the group consisting of monohydric alcohol, water, diol or diamine in a ratio of 3:1 respectively so that on average, one of the isocyanate groups is reacted,
(b) reacting the isocyanurate obtained in step (a) with at least one compound to form a carbamate containing compound having the formula 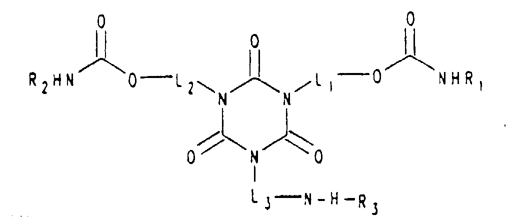 wherein
L₁, L₂, and L₃ each independently represents a linking group, and
R₁, and R₂ each independently represents H, alkyl, or cycloalkyl and R₃ is a residue resulting from the reaction of isocyanate and water, monohydric alcohol, diamine or diol
(c) reacting component (b) with a component having a plurality of functional groups that are reactive with carbamate.

2. A coating composition according to claim 1, wherein component (a) is reacted with a compound containing an isocyanate reactive group and a carbamate group to, form component (b).

3. A coating composition according to either of the claims 1 and 2, wherein component (a) is reacted with a compound having a hydroxy group and a cylic carbonate group, followed by reaction with ammonia, to form component (b).

4. A coating composition according to any of the claims 1 to 3, wherein R1 and R2 each independently represents H.

5. A coating composition according to any of the claims 1 to 4, where R₁ and R₂ each independently represents alkyl, cycloalkyl or aryl.

6. A coating composition according to any of the claims 1 to 5, wherein L₁., L₂, and L₃ each includes a urethane linkage.

7. A coating composition according to any of the claims 1 to 6, wherein the functional groups an the component (c) are selected from the group consisting of active methylol or methylalkyoxy groups, cyclic carbonate groups, anhydride groups, and siloxane groups.

8. A coating composition according to any of the claims 1 to 7, wherein component (c) is an aminoplast resin.

9. A coating composition according to claim 8 wherein the aminoplast resin is melamine formaldehyde or alkylated melamine formaldehyde.

10. A coating composition according to any of the claims 1 to 7, wherein component (c) is a polymer or oligomer backbone having said carbamate-reactive functional groups appended thereto.

11. A coating composition according to claim 10 wherein said polymeric or oligomeric backbone is derived from acrylic or methacrylic monomers.

12. A coating composition according to any of the claims 1 to 11, wherein component (b) has the formula: 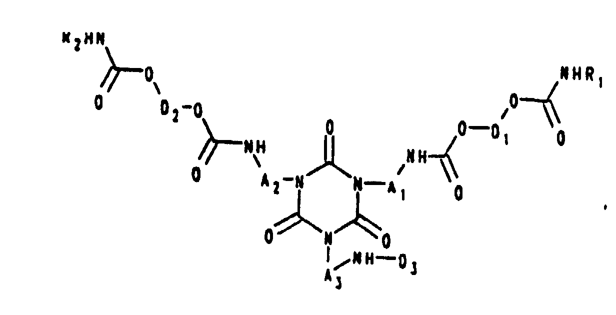 wherein
A₁, A₂ and A₃ each independently represents a divalent linking group, and
D₁ and D₂ each independently represents a divalent linking group and D₃ represents the residue resulting from the reaction of isocyanate and water, monohydric alcohol, diol or diamine.

13. A composite color-plus-clear coating comprising a colored base coating and a clear coating, said clear coating derived from a coating composition according to the claims 1 or 4.

14. A composite color-plus-clear coating comprising a colored base coating and a clear coating, said clear coating derived from a coating composition according to the claims 1 or 4, wherein the component (b) has the formula 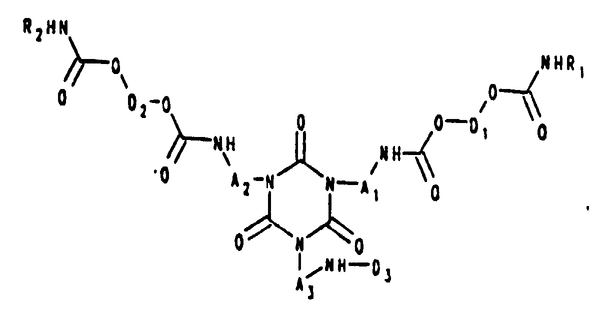 wherein
A₁, A₂, and A₃ each independently represents a divalent linking group, and
D₁ and D₂ each independently represents a divalent linking group and D₃ represents the residue resulting from the reaction of isocyanate and water, monohydric alcohol, diol or diamine.

## Patentansprüche

1. Beschichtungszusammensetzung, erhältlich dadurch, daß man
(a) ein tri-NCO-funktionelles Isocyanurat mit einer Verbindung aus der Gruppe bestehend aus einwertigem Alkohol, Wasser, Diol oder Diamin in einem Verhältnis von 3:1 bzw. so, daß im Durchschnitt eine der Isocyanatgruppen abreagiert, umsetzt,
(b) das in Schritt (a) erhaltene Isocyanurat mit mindestens einer Verbindung zu einer carbamatgruppen-haltigen Verbindung der Formel 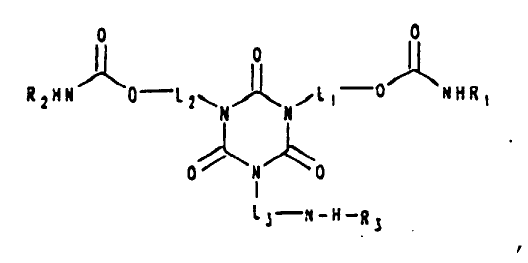 worin
L₁, L₂ und L₃ unabhängig voneinander jeweils für eine Brückengruppe und
R₁ und R₂ unabhängig voneinander jeweils für H, Alkyl oder Cycloalkyl stehen und R₃ für einen Molekülrest, der sich aus der Umsetzung von Isocyanat und Wasser, einwertigem Alkohol, Diamin oder Diol ergibt, steht, umsetzt und
(c) die Komponente (b) mit einer Komponente mit mehreren gegenüber Carbamat reaktiven Gruppen umsetzt.

2. Beschichtungszusammensetzung nach Anspruch 1, wobei die Komponente (a) mit einer eine gegenüber Isocyanat reaktive Gruppe und eine Carbamatgruppe enthaltenden Verbindung zu Komponente (b) umgesetzt wird.

3. Beschichtungszusammensetzung nach Anspruch 1 oder 2, wobei die Komponente (a) durch Umsetzung mit einer Verbindung mit einer Hydroxylgruppe und einer cyclischen Carbonatgruppe und anschließende Umsetzung mit Ammoniak in Komponente (b) überführt wird.

4. Beschichtungszusammensetzung nach einem der Ansprüche 1 bis 3, wobei R₁ und R2 unabhängig voneinander jeweils für H stehen.

5. Beschichtungszusammensetzung nach einem der Ansprüche 1 bis 4, wobei R₁ und R₂ unabhängig voneinander jeweils für Alkyl, Cycloalkyl oder Aryl stehen.

6. Beschichtungszusammensetzung nach einem der Ansprüche 1 bis 5, wobei L₁, L₂ und L₃ jeweils eine Urethanbrücke enthalten.

7. Beschichtungszusammensetzung nach einem der Ansprüche 1 bis 6, wobei die funktionellen Gruppen der Komponente (c) aus der Gruppe bestehend aus aktiven Methylol- oder Methylalkoxygruppen, cyclischen Carbonatgruppen, Anhydridgruppen und Siloxangruppen stammen.

8. Beschichtungszusammensetzung nach einem der Ansprüche 1 bis 7, wobei es sich bei der Komponente (c) um ein Aminoplastharz handelt.

9. Beschichtungszusammensetzung nach Anspruch 8, wobei es sich bei dem Aminoplastharz um ein Melamin-Formaldehyd-Harz oder ein alkyliertes Melamin-Formaldehyd-Harz handelt.

10. Beschichtungszusammensetzung nach einem der Ansprüche 1 bis 7, wobei es sich bei der Komponente (c) um eine Polymer- oder Oligomerhauptkette mit daran gebundenen, gegenüber Carbamat reaktiven funktionellen Gruppen handelt.

11. Beschichtungszusammensetzung nach Anspruch 10, wobei die Polymer- oder Oligomerhauptkette sich von Acryl- oder Methacrylmonomeren ableitet.

12. Beschichtungszusammensetzung nach einem der Ansprüche 1 bis 11, wobei Komponente (b) die Formel: 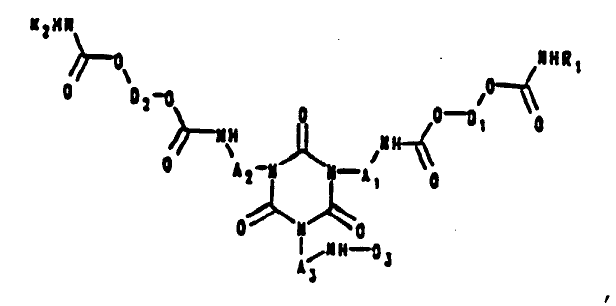 worin
A₁, A₂ und A₃ unabhängig voneinander jeweils für eine zweiwertige Brückengruppe und
D₁ und D₂ unabhängig voneinander jeweils für eine zweiwertige Brückengruppe stehen und R₃ für einen Molekülrest, der sich aus der Umsetzung von Isocyanat und Wasser, einwertigem Alkohol, Diol oder Diamin ergibt, steht, aufweist.

13. Farblack-Klarlack-Verbundbeschichtung, enthaltend einen farbigen Basislack und einen Klarlack, der sich von einer Beschichtungszusammensetzung gemäß einem der Ansprüche 1 oder 4 ableitet.

14. Farblack-Klarlack-Verbundbeschichtung, enthaltend einen farbigen Basislack und einen Klarlack, der sich von einer Beschichtungszusammensetzung gemäß einem der Ansprüche 1 oder 4 ableitet, wobei die Komponente (b) die Formel 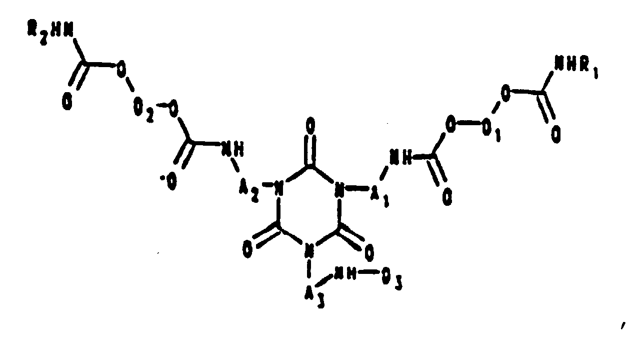 worin
A₁, A₂ und A₃ unabhängig voneinander jeweils für eine zweiwertige Brückengruppe und
D₁ und D₂ unabhängig voneinander jeweils für eine zweiwertige Brückengruppe stehen und R₃ für einen Molekülrest, der sich aus der Umsetzung von Isocyanat und Wasser, einwertigem Alkohol, Diol oder Diamin ergibt, steht, aufweist.

## Revendications

1. Composition de revêtement obtenue par le procédé de :
(a) réaction d'un isocyanurate à trois fonctions NCO avec un composé choisi parmi le groupe constitué d'un alcool monohydrique, de l'eau, d'un diol ou d'une diamine en un rapport de 3:1 respectivement, de telle sorte qu'en moyenne, un des groupes isocyanate réagisse,
(b) réaction de l'isocyanurate obtenu à l'étape (a) avec au moins un composé pour former un composé renfermant un carbamate, de formule 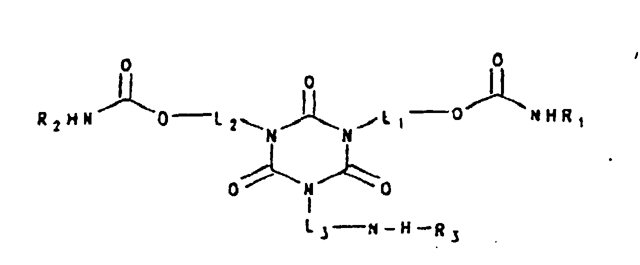 dans laquelle
L₁, L₂ et L₃ représentent chacun indépendamment un groupe de liaison, et
R₁ et R₂ représentent chacun indépendamment H, un alkyle ou un cycloalkyle et R₃ est un radical résultant de la réaction d'un isocyanate et de l'eau, d'un alcool monohydrique, d'une diamine ou d'un diol
(c) réaction du composant (b) avec un composant ayant une pluralité de groupes fonctionnels qui sont réactifs vis-à-vis d'un carbamate;

2. Composition de revêtement selon la revendication 1, dans laquelle le composant (a) est mis à réagir avec un composé renfermant un groupe réactif vis-à-vis d'un isocyanate et un groupe carbamate pour former le composant (b).

3. Composition de revêtement selon l'une ou l'autre des revendications 1 et 2, dans laquelle le composant (a) est mis à réagir avec un composé renfermant un groupe hydroxy et un groupe carbonate cyclique, suivi d'une réaction avec l'ammoniac, pour former le composant (b).

4. Composition de revêtement selon l'une quelconque des revendications 1 à 3, dans laquelle R₁ et R₂ représentent chacun indépendamment H.

5. Composition de revêtement selon l'une quelconque des revendications 1 à 4, dans laquelle R₁ et R2 représentent chacun indépendamment un alkyle, un cycloalkyle ou un aryle.

6. Composition de revêtement selon l'une quelconque des revendications 1 à 5, dans laquelle L₁, L₂ et L₃ renferment chacun une liaison uréthanne.

7. Composition de revêtement selon l'une quelconque des revendications 1 à 6, dans laquelle les groupes fonctionnels sur le composant (c) sont choisis parmi le groupe constitué de groupes méthylalcoxy ou méthylol actifs, de groupes carbonate cycliques, de groupes anhydride et de groupes siloxane.

8. Composition de revêtement selon l'une quelconque des revendications 1 à 7, dans laquelle le composant (c) est une résine aminoplaste.

9. Composition de revêtement selon la revendication 8, dans laquelle la résine aminoplaste est une résine mélamine-formaldéhyde ou mélamine-formaldéhyde alkylée.

10. Composition de revêtement selon l'une quelconque des revendications 1 à 7, dans laquelle le composant (c) est un tronc polymère ou oligomère auquel sont attachés lesdits groupes fonctionnels réactifs vis-à-vis d'un carbamate.

11. Composition de revêtement selon la revendication 10, dans laquelle ledit tronc polymère ou oligomère est dérivé de monomères acryliques ou méthacryliques.

12. Composition de revêtement selon l'une quelconque des revendications 1 à 11, dans laquelle le composant (b) est de formule : 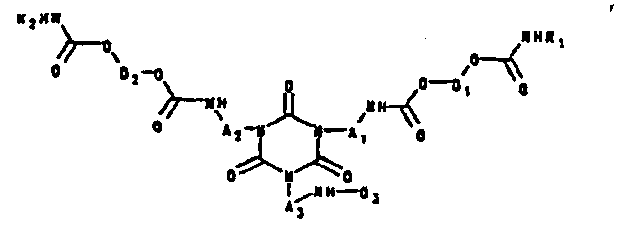 dans laquelle
A₁, A₂ et A₃ représentent chacun indépendamment un groupe de liaison divalent, et D₁ et D₂ représentent chacun un groupe de liaison divalent et D₃ représente le radical résultant de la réaction d'un isocyanate, et de l'eau, d'un alcoolmonohydrique, d'un diol ou d'une diamine.

13. Revêtement composite couleur-plus-transparence comprenant un revêtement de fond coloré et un revêtement transparent, ledit revêtement transparent provenant d'une composition de revêtement selon les revendications 1 ou 4.

14. Revêtement composite couleur-plus-transparence comprenant un revêtement de fond coloré et un revêtement transparent, ledit revêtement transparent provenant d'une composition de revêtement selon les revendications 1 ou 4, dans lequel le composant (b) est de formule 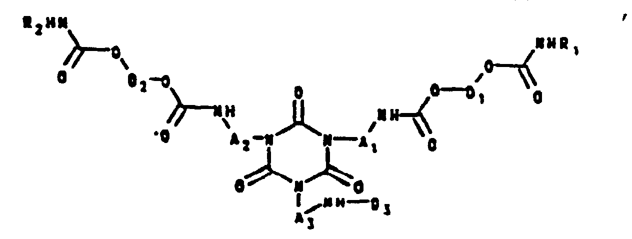 dans laquelle
A₁, A₂ et A₃ représentent chacun indépendamment un groupe de liaison divalent, et D₁ et D₂ représentent chacun un groupe de liaison divalent et D₃ représente le radical résultant de la réaction d'un isocyanate, et de l'eau, d'un alcoolmonohydrique, d'un diol ou d'une diamine.
